(19) Europäisches Patentamt
European Patent Office
Office européen des brevets

(11)  **EP 3 446 674 A1**

(12)  **EUROPEAN PATENT APPLICATION**

(43) Date of publication:
   27.02.2019 Bulletin 2019/09

(51) Int Cl.:
   *A61K 6/083* (2006.01)   *A61K 6/00* (2006.01)

(21) Application number: 18199925.1

(22) Date of filing: 17.09.2013

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**

(30) Priority: 27.09.2012 JP 2012214038

(62) Document number(s) of the earlier application(s) in
   accordance with Art. 76 EPC:
   **13842387.6 / 2 902 007**

(71) Applicant: **Tokuyama Dental Corporation
   Tokyo 110-0016 (JP)**

(72) Inventors:
   • **TORIYABE, Chika**
    **Taito-ku, Tokyo 110-0016 (JP)**
   • **AKIZUMI, Hironobu**
    **Taito-ku, Tokyo 110-0016 (JP)**

(74) Representative: **J A Kemp
   14 South Square
   Gray's Inn
   London WC1R 5JJ (GB)**

Remarks:
   This application was filed on 11.10.2018 as a
   divisional application to the application mentioned
   under INID code 62.

(54)  **DENTAL FILLING  REPAIRING MATERIAL**

(57)   A dental restorative material including a photocurable composition that contains a polymerizable monomer component (A), an inorganic filler component (B) having an average particle size of not smaller than 0.07 $\mu$m, and a photo polymerization initiator (C),
the inorganic filler component (B) being contained in an amount of 100 to 1500 parts by mass per 100 parts by mass of the polymerizable monomer component (A), and
the polymerizable monomer component (A) and the inorganic filler component (B) being so selected as to satisfy a condition (X1) represented by the following formulas (1a) and (1b):

$$nF - 0.005 < nM < nF + 0.005 \qquad (1a)$$

$$nF + 0.020 < nP < nF + 0.040 \qquad (1b)$$

wherein,
nM is a refractive index of the polymerizable monomer component (A) at 25°C,
nP is a refractive index at 25°C of a polymer obtained by polymerizing the polymerizable monomer component (A), and
nF is a refractive index of the inorganic filler component (B) at 25°C.

EP 3 446 674 A1

**Description**

Technical Field:

This invention relates to a dental restorative material and, more specifically, to a dental restorative material used for restoring deep cavities formed in the molars (posterior teeth).

Background Art:

Owing to their capability of imparting a color tone similar to that of natural teeth and easiness of use for treatment, dental restorative materials are now finding a use that is widely spreading in recent years and have now been applied to most of the treatment of the front teeth. Further, some of the dental restorative materials that have now been developed feature excellent mechanical strength, and can also be used for restoring the molars (posterior teeth) that receive large occlusive pressures.

As the restorative materials, there have been advantageously used photocurable compositions obtained by blending a polymerizable monomer with large amounts of an inorganic filler and a photo polymerization initiator. For instance, a dental adhesive material is applied to the cavity of a tooth that is to be restored, and the restorative material (photocurable composition) is filled in the cavity and is formed in the shape of the tooth followed by the irradiation with activating light by using a dental curing light so as to be polymerized and cured to thereby restore the tooth using the polymerized and cured material that is formed.

Another practice is that in a dental laboratory, a photocurable restorative material is applied on a gypsum model in the shape of a tooth that is to be restored and is polymerized and cured by the irradiation with light. Next, in a dental clinic, the thus obtained restorative material that has been cured is adhered to the tooth by using a dental cement to thereby restore the tooth. The dental restorative material is photocured by using the visible light from the standpoint of safety to the human body. For this purpose, further, a visible light polymerization initiator is, usually, used as the photo polymerization initiator.

In restoring the teeth in recent years, what is regarded particularly important is the concept of minimal intervention, i.e., the idea of lengthening the life of the natural teeth as much as possible by grinding the teeth as little as possible. The above concept has now been generally accepted owing primarily to such a background that the adhesion of the dental adhesive material to the teeth has now been so improved that the teeth can now be treated without the need of grinding the teeth to an excess degree. In such a background, the dental restorative material (called composite resin) is now finding an ever increasing usefulness in the clinical sites.

As the composite resin is now being used ever widely, more attention has now been paid to simplifying the operation for using the composite resin from the standpoint of reducing burden to the dentist and the patient and reducing technique sensitivity by the dentist. For instance, there has been developed a system reducing the number of the steps for applying the dental adhesive material.

To restore a large cavity (a cavity of a depth of 3 to 6 mm formed in, for example, a posterior tooth) by using the composite resin, there is, usually, employed means that repeats the operation of applying a photocurable composition that is the composite resin onto the cavity in a thickness of about 1 to about 2 mm and irradiating the composite resin with light to polymerize and cure it. This is because if the photocurable composition is filled at one time in a large cavity and is polymerized by the irradiation with light, then the composition is likely to peel on the bottom or at the margin due to contraction by polymerization or due to defective polymerization and causing defect such as developing color along the margin and secondary caries after the treatment.

On the other hand, means that applies the photocurable composition onto the cavity little by little and cures the composition by polymerization every time, is not only cumbersome but may also entrap air bubbles between the layer that is polymerized and cured first and the layer that is polymerized and cured next. The bubbles that are entrapped not only cause a decrease in the life of the polymerized and cured body (composite resin) due to decreased strength thereof but also cause the secondary caries.

In order to solve the above problems, patent documents 1 to 4 are proposing photocurable compositions decreasing the contraction by polymerization (contraction factor and stress of contraction). The photocurable compositions that lowly contract by polymerization can be filled in one time in a deep cavity formed in the posterior tooth and can be cured up to the bottom portion and to the edges without forming gap by the irradiation with light of only one time.

Further, a patent document 5 is proposing a photocurable composition using an inorganic filler that is so selected as to satisfy such a condition that a refractive index (nF) of the inorganic oxide (inorganic filler) is a value lying between a value higher by 0.005 than a refractive index (nM) of a polymerizable monomer and a value lower by 0.005 than a refractive index (nP) of a polymer obtained by polymerizing the polymerizable monomer. The above photocurable composition can be deeply cured. Therefore, by using the above photocurable composition for restoring a deep cavity formed in the posterior tooth, the restoration can be done by, for example, filling and photo-curing the resin only one time.

However, though the above conventional photocurable composition could improve the efficiency of handling for restoring the cavity, there still remained a problem in that the appearance of the cured body (composite resin) restoring the cavity was not in match with the teeth.

Prior Art Documents:

Patent Documents:

Patent document 1: JP-T-2009-540107
Patent document 2: JP-T-2008-502697
Patent document 3: JP-T-2004-527602
Patent document 4: JP-A-2004-149587
Patent document 5: JP-A-62-86003

Outline of the Invention:

Problems that the Invention is to Solve:

It is, therefore, an object of the present invention to provide a dental restorative material that can be cured deep, can be favorably worked for restoring the cavities, can be filled in even a deep cavity such as the one formed in a posterior tooth in one time or in a small number of times and can be effectively polymerized and cured up to the bottom portion thereof by the irradiation with visible light exhibiting appearance in match with that of the natural teeth.

Means for Solving the Problems:

To achieve the above object, the present inventors have keenly forwarded the study. As a result, the inventors have discovered the fact that the above object can be achieved if an inorganic filler is used in combination with a polymer obtained from a polymerizable monomer, the inorganic filler being the one having a refractive index that is in a range close to that of the polymerizable monomer but is suitably separated away from the refractive index of the polymer that is obtained from the polymerizable monomer, and have completed the present invention.

According to the present invention, there is provided a dental restorative material including a photocurable composition that contains a polymerizable monomer component (A), an inorganic filler component (B) having an average particle size of not smaller than 0.07 $\mu$m, and a photo polymerization initiator (C),

the inorganic filler component (B) being contained in an amount of 100 to 1500 parts by mass per 100 parts by mass of the polymerizable monomer component (A), and

the polymerizable monomer component (A) and the inorganic filler component (B) being so selected as to satisfy a condition (X1) represented by the following formulas (1a) and (1b):

$$nF - 0.005 < nM < nF + 0.005 \qquad (1a)$$

$$nF + 0.020 < nP < nF + 0.040 \qquad (1b)$$

wherein,

nM is a refractive index of the polymerizable monomer component (A) at 25°C,
nP is a refractive index at 25°C of a polymer obtained by polymerizing the polymerizable monomer component (A), and
nF is a refractive index of the inorganic filler component (B) at 25°C.

In the dental restorative material of the present invention (hereinafter often referred to simply as "dental restorative material"), it is desired that:

(1) The polymerizable monomer component (A) and the inorganic filler component (B) are so selected as to satisfy a condition (X2) represented by the following formulas (2a) and (2b):

$$nF - 0.005 < nM < nF + 0.003 \qquad (2a)$$

$$nF + 0.025 < nP < nF + 0.035 \qquad (2b)$$

wherein, nM, nP and nF are as defined above;

(2) The polymerizable monomer component (A) contains a plurality of kinds of polyfunctional (meth)acrylic compounds and has a refractive index (at 25°C) in a range of 1.48 to 1.55;

(3) The plurality of kinds of polyfunctional (meth)acrylic compounds include a combination of polyfunctional aromatic (meth)acrylates and polyfunctional aliphatic (meth)acrylates;

(4) The polyfunctional aromatic (meth)acrylate is a 2,2-bis[(3-methacryloyloxy-2-hydroxypropyloxy)phenyl] propane and/or a 2,2-bis[(4-methacryloyloxypolyethoxyphenyl)] propane, and the polyfunctional aliphatic (meth)acrylate is a triethylene glycol dimethacrylate and/or a 1,6-bis (methacrylethyloxycarbonylamino) trimethylhexane;

(5) A silica composite oxide is used as the inorganic filler, and the content of the silica component in the composite oxide is so set as to satisfy the condition (X1) or (X2);

(6) The depth of curing is not less than 6 mm as measured after having been irradiated with light of a light quantity of 500 mW/cm$^2$ for 30 seconds by using a halogen-type dental curing light;

(7) The dental restorative material, further, contains a coloring agent (D) and has a contrast ratio of not more than 0.30 as measured in an uncured state having a thickness of 1 mm and a contrast ratio of not more than 0.55 as measured in a state of a cured body having a thickness of 1 mm; and

(8) The dental restorative material is used for restoring cavities formed in the posterior teeth.

In the present invention, the refractive index of the polymer obtained by polymerizing the polymerizable monomer component (A) stands for a value of the polymer of a thickness of 0.5 mm obtained by cast-polymerizing the polymerizable monomer component (A) under a predetermined condition (nearly the same as the condition for polymerization in a cavity) as measured by using the Abbe's refractometer. That is, if there is used only one kind of the polymerizable monomer component (A), then the value is a refractive index of a homopolymer of the polymerizable monomer. If there are used a plurality of kinds of the polymerizable monomer components (A), the value is a refractive index of a random copolymer of the plurality of the polymerizable monomers. The condition for polymerization has been set to be the same as that of when a cavity in the posterior tooth is to be restored.

Here, the refractive index is a value at 25°C unless stated otherwise.

Effects of the Invention:

The dental restorative material (photocurable composition) of the present invention features a high loading of the inorganic filler and excellently reduces the contraction by polymerization.

Further, the dental restorative material permits light to pass through excellently in the region of visible light and can be deeply cured. Therefore, even a deep and large cavity formed in the posterior tooth can be restored by filling it and photopolymerizing it only one time or by repeating the filling and photo polymerization a small number of times.

Besides, the cured body of the dental restorative material (photocurable composition) is translucent close to the natural teeth, has appearance in match with the appearance of the natural teeth and, therefore, makes it possible to restore the cavity formed in the posterior tooth without impairing the esthetics.

Modes for Carrying Out the Invention:

The photocurable composition of the present invention used as the dental restorative material is filled in a cavity that is formed in a tooth due to decaying, and is photo-cured to restore the tooth. The photocurable composition contains the polymerizable monomer component (A), the inorganic filler component (B) and the photo polymerization initiator (C) as essential components. In addition to these essential components, the photocurable composition of the invention, further, contains the coloring agent (D) and any other components. Here, the polymerizable monomer component (A) and the inorganic filler component (B) have been so selected as to satisfy a specific condition.

The individual components will be described below in detail.

<Polymerizable monomer component (A)>

The polymerizable monomer that can be used as one of the components of the dental restorative material (photocurable composition) of the present invention, is an organic compound that has a polymerizable group and can be polymerized with a photo polymerization initiator and, specifically, is an organic compound that is capable of forming a polymer having high transparency and having a total light transmittance of not less than 85% and, preferably, not less than 90%.

Representative examples of the polymerizable monomer include cationically polymerizable monomers and radically polymerizable monomers.

Representative examples of the cationically polymerizable monomer include vinyl ether compound, epoxy compound, oxetane compound, cyclic ether compound, bicyclic orthoester compound, cyclic acetal compound, bicyclic acetal compound and cyclic carbonate compound.

Further, a representative example of the radically polymerizable monomer is a (meth)acrylic compound.

In the present invention, the (meth) acrylic compound is best suited as the polymerizable monomer component (A) from the standpoint of, specifically, low cytotocixity and high polymerizing activity.

In the invention, the (meth)acrylic compounds can be divided into monofunctional compounds and polyfunctional compounds having two or more functions (e.g., having two functions, three functions and four functions). Their suitable examples are as described below.

(A1) Monofunctional (meth)acrylic compounds:

The monofunctional methacrylic compounds can be divided into those having neither an acid group nor a hydroxyl group, those having an acid group, and those having a hydroxyl group.

(1) Monofunctional (meth)acrylic compounds having neither an acid group nor a hydroxyl group.

Methyl (meth)acrylate,
Ethyl (meth)acrylate,
n-Butyl (meth)acrylate,
2-Ethylhexyl (meth)acrylate,
n-Lauryl (meth)acrylate,
n-Stearyl (meth)acrylate,
Tetrafurfuryl (meth)acrylate,
Glycidyl (meth)acrylate,
Methoxyethylene glycol (meth)acrylate,
Methoxydiethylene glycol (meth)acrylate,
Methoxytriethylene glycol (meth)acrylate,
Methoxypolyethylene glycol (meth)acrylate,
Ethoxyethylene glycol (meth)acrylate,
Ethoxydiethylene glycol (meth)acrylate,
Ethoxytriethylene glycol (meth)acrylate,
Ethoxypolyethylene glycol (meth)acrylate,
Phenoxyethylene glycol (meth)acrylate,
Phenoxydiethylene glycol (meth)acrylate,
Phenoxytriethylene glycol (meth)acrylate,
Phenoxypolyethylene glycol (meth)acrylate,
Cyclohexyl (meth)acrylate,
Benzyl (meth)acrylate,
Isoboronyl (meth)acrylate, and
Trifluoroethyl (meth)acrylate.

(2) Monofunctional (meth)acrylic compounds having an acid group.

(Meth) acrylic acid,
N-(Meth)acryloyl glycine,
N-(Meth)acryloylaspartic acid,
N-(Meth)acryloyl-5-aminosalicylic acid,
2-(Meth)acryloyloxyethylhydrogen succinate,
2-(Meth)acryloyloxyethylhydrogen phthalate,
2-(Meth)acryloyloxyethylhydrogen maleate,
6-(Meth)acryloyloxyethylnaphthalene-1,2,6-tricarboxylic acid,
O-(Meth)acryloyl tyrosine,

N-(Meth)acryloyl tyrosine,
N-(Meth)acryloylphenyl alanine,
N-(Meth)acryloyl-p-aminobenzoic acid,
N-(Meth)acryloyl-o-aminobenzoic acid,
p-Vinylbenzoic acid,
2-(Meth)acryloyloxybenzoic acid,
3-(Meth)acryloyloxybenzoic acid,
4-(Meth)acryloyloxybenzoic acid,
N-(Meth)acryloyl-5-aminosalicylic acid,
N-(Meth)acryloyl-4-aminosalicylic acid, and
Acid anhydrides corresponding to the above carboxyl group-containing compounds.
11-(Meth)acryloyloxyundecane-1,1-dicarboxylic acid,
10-(Meth)acryloyloxydecane-1,1-dicarboxylic acid,
12-(Meth)acryloyloxydodecane-1,1-dicarboxylic acid,
6-(Meth)acryloyoxyhexane-1,1-dicarboxylic acid,
2-(Meth)acryloyloxyethyl-3'-methacryloyloxy-2'-(3,4-dicarboxybenzoyloxy)propyl succinate,
4-(2-(Meth)acryloyloxyethyl) trimeritate anhydride,
4-(2-(Meth)acryloyloxyethyl) trimeritate,
4-(Meth)acryloyloxyethyl trimeritate,
4-(Meth)acryloyloxybutyl trimeritate,
4-(Meth)acryloyloxyhexyl trimeritate,
4-(Meth)acryloyloxydecyl trimeritate,
4-(Meth)acryloyloxybutyl trimeritate,
6-(Meth)acryloyloxyethylnaphthalene-1,2,6-tricarboxylic acid anhydride,
6-(Meth)acryloyloxyethylnaphthalene-2,3,6-tricarboxylic acid anhydride,
4-(Meth)acryloyloxyethylcarbonylpropionoyl-1,8-naphthalic acid anhydride,
4-(Meth)acryloyloxyethylnaphthalene-1,8-tricarboxylic acid anhydride,
9-(Meth)acryloyloxynonane-1,1-dicarboxylic acid,
13-(Meth)acryloyloxytridecane-1,1-dicarboxylic acid,
11-(Meth)acrylamideundecane-1,1-dicarboxylic acid,
2-(Meth)acryloyloxyethyldihydrogen phosphate,
2-(Meth)acryloyloxyethylphenylhydrogen phosphate,
10-(Meth)acryloyloxydecyldihydrogen phosphate,
6-(Meth)acryloyloxyhexyldihydrogen phosphate,
2-(Meth)acryloyloxyethyl-2-bromoethylhydrogen phosphate,
2-(Meth)acrylamidoethyldihydrogen phosphate,
2-(Meth)acrylamide-2-methylpropanesulfonic acid,
10-Sulfodecyl (meth)acrylate,
3-(Meth)acryloxypropyl-3-phosphonopropionate,
3-(Meth)acryloxypropylphosphonoacetate,
4-(Meth)acryloxybutyl-3-phosphonopropionate,
4-(Meth)acryloxybutylphosphonoacetate,
5-(Meth)acryloxypentyl-3-phosphonopropionate,
5-(Meth)acryloxypentylphosphonoacetate,
6-(Meth)acryloxyhexyl-3-phosphonopropionate,
6-(Meth)acryloxyhexylphosphonoacetate,
10-(Meth)acryloxydecyl-3-phosphonopropionate,
10-(Meth)acryloxydecylphosphonoacetate,
2-(Meth)acryloxyethylphenyl phosphate,
2-(Meth)acryloyloxyethylphosphonic acid,
10-(Meth)acryloyloxydecylphosphonic acid,
N-(Meth)acryloyl-$\omega$-aminopropylphosphonic acid,
2-(Meth)acryloyloxyethylphenylhydrogen phosphate,
2-(Meth)acryloyloxyethyl-2'-bromoethylhydrogen phosphate, and
2-(Meth)acryloyloxyethylphenyl phosphonate.

(3) Monofunctional (meth)acrylic compounds having a hydroxyl group.

2-Hydroxyethyl (meth)acrylate,
3-Hydroxypropyl (meth)acrylate,
4-Hydroxybutyl (meth)acrylate,
6-Hydroxyhexyl (meth)acrylate,
10-Hydroxydecyl (meth)acrylate,
Propylene glycol mono(meth)acrylate,
Glycerol mono(meth)acrylate,
Erythritol mono(meth)acrylate,
N-Methylol (meth)acrylamide,
N-Hydroxyethyl (meth)acrylamide, and
N,N-(Dihydroxyethyl) (meth)acrylamide.

(A2) Bifunctional (meth)acrylic compounds:

The bifunctional (meth) acrylic compounds can be roughly divided into those having an aromatic group and those of the aliphatic type without aromatic group.

(1) Aromatic bifunctional (meth)acrylic compounds.

2,2-Bis(methacryloyloxyphenyl) propane,
2,2-Bis(methacryloylethoxyphenyl) propane,
2,2-Bis[4-(3-methacryloyloxy)-2-hydroxypropoxyphenyl) propane,
2,2-Bis(4-methacryloyloxyphenyl) propane,
2,2-Bis(4-methacryloyloxypolyethoxyphenyl) propane,
2,2-Bis(4-methacryloyloxydiethoxyphenyl) propane,
2,2-Bis(4-methacryloyloxytetraethoxyphenyl) propane,
2,2-Bis(4-methacryloyloxypentaethoxyphenyl) propane,
2,2-Bis(4-methacryloyloxydipropoxyphenyl) propane,
2(4-Methacryloyloxydiethoxyphenyl)-2(4-methacryloyloxydiethoxyphenyl) propane,
2(4-Methacryloyloxydiethoxyphenyl)-2(4-methacryloyloxytriethoxyphenyl) propane,
2(4-Methacryloyloxydipropoxyphenyl)-2(4-methacryloyloxytriethoxyphenyl) propane,
2,2-Bis(4-methacryloyloxypropoxyphenyl) propane,
2,2-Bis(4-methacryloyloxyisopropoxyphenyl) propane,
Acrylic compounds corresponding to the above methacrylic compounds, and
Diadducts obtained by adding a methacrylate or an acrylate having an OH group to the diisocyanate compounds having an aromatic group.

Here, representative examples of the methacrylate having an OH group include 2-hydroxyethyl methacrylate, 2-hydroxypropyl methacrylate and 3-chloro-2-hydroxypropyl methacrylate while representative examples of the diisocyanate include diisocyanate methylbenzene and 4,4'-diphenolmethane diisocyanate.

(2) Aliphatic bifunctional (meth)acrylic compounds:

Ethylene glycol dimethacrylate,
Diethylene glycol dimethacrylate,
Triethylene glycol dimethacrylate,
Tetraethylene glycol dimethacrylate,
Neopentyl glycol dimethacrylate,
1,3-Butanediol dimethacrylate,
1,4-Butanediol dimethacrylate,
1,6-Hexanediol dimethacrylate,
1,2-Bis(3-methacryloyloxy-2-hydroxypropoxy) ethyl,
1,6-Bis(methacrylethyloxycarbonylamino) trimethylhexane,
Acrylates corresponding to the above methacrylates, and
Diadducts obtained by adding the methacrylate or the acrylate having an OH group to the aliphatic diisocyanate compounds.

Here, representative examples of the aliphatic diisocyanate include hexamethylene diisocyanate, trimethylhexamethylene diisocyanate, diisocyanatemethyl cyclohexane, isophorone diisocyanate and methylene bis(4-cyclohexyl isocyanate) while examples of the methacrylate having an OH group include 2-hydroxyethyl methacrylate, 2-hydroxypropyl methacrylate and 3-chloro-2-hydroxypropyl methacrylate.

Further, some of the aliphatic bifunctional (meth)acrylic compounds include an acid group, such as those described below.

Acrylic anhydride,

Methacrylic anhydride,

1,2-Bis(3-methacryloyloxy-2-hydroxypropoxy) ethyl,

Di(2-methacryloyloxypropyl) phosphate,

Di[2-(meth)acryloyloxyethyl] hydrogen phosphate,

Di[4-(meth)acryloyloxybutyl] hydrogen phosphate,

Di[6-(meth)acryloyloxyhexyl] hydrogen phosphate,

Di[8-(meth)acryloyloxyoctyl] hydrogen phosphate,

Di[9-(meth)acryloyloxynonyl] hydrogen phosphate,

Di[10-(meth)acryloyloxydecyl] hydrogen phosphate, and

1,3-Di(meth)acryloyloxypropyl-2-dihydrogen phosphate.

(A3) Trifunctional (meth)acrylic compounds:

Trimethylolpropane tri(meth)acrylate,

Trimethylolethane tri(meth)acrylate,

Pentaerythritol tri(meth)acrylate,

Dipentaerythritol tri(meth)acrylate,

Ethoxylated trimethylolpropane tri(meth)acrylate,

Propoxylated trimethylolpropane tri (meth) acrylate, and

Tris(2-(meth)acryloxyethyl isocyanuate).

(A4) Tetrafunctional (meth)acrylic compounds:

Pentaerythritol tetra(meth)acrylate,

Ethoxylated pentaerythritol tetra(meth)acrylate,

Propoxylated pentaerythritol tetra(meth)acrylate, and

Ethoxylated ditrimethylolpropane tetra(meth)acrylate.

In the present invention, at least any one of the above-mentioned polymerizable monomers is selected and is used as the polymerizable monomer component (A) so as to satisfy the condition (X1) and, preferably, the condition (X2) that will be described later. Here, however, it is desired to use a plurality of kinds of polyfunctional polymerizable monomers (e.g., polyfunctional (meth)acrylic compounds described above) having at least two or more polymerizable groups from the standpoint of improving mechanical properties (e.g., strength and water-resisting property) of the cured body that is formed and adhering property to the teeth. That is, despite the polymerizable monomers are selected so as to satisfy the condition (X1) or (X2) that will be described later, the cured body that is formed has a low mechanical strength and fails to satisfy properties as the restorative if the polymerizable monomers that are used are all monofunctional.

Usually, it is desired that not less than 60% by mass and, specifically, not less than 70% by mass of the polymerizable monomer components (A) are the polyfunctional polymerizable monomers.

In the present invention, it is also allowable to use polymerizable monomers having photofragmenting property as disclosed in, for example, JP-T-2009-540107 and WO2007/146239 as well as polymerizable macrocyclic oligomers as disclosed in JP-T-2008-502697 in addition to using the above-mentioned various polymerizable monomers. Such polymerizable monomers are effective in, specifically, suppressing the contraction by polymerization.

<Inorganic filler components (B)>

As the inorganic filler component (B), there can be used any inorganic fillers that have been known in the field of dental curable compositions so far as they are so selected as to satisfy the condition (X1) and, preferably, the condition (X2) that will be described later. Here, however, it is necessary that they have an average particle size of not less than 0.07 $\mu$m. This is because with the average particle size being smaller than 0.07 $\mu$m, the particle size becomes smaller than the wavelengths of the visible light, and the inorganic filler components become transparent irrespective of their refractive indexes. Therefore, it becomes difficult to obtain a cured body having desired appearance in match with that of the natural teeth.

It is, further, desired that the inorganic filler component has an average particle size which is not larger than 100 $\mu$m and, specifically, in a range of not larger than 5 $\mu$ m from such a standpoint that it is homogeneously dispersed in the curable composition and that the cured body that is obtained has a high surface gloss obtaining esthetics.

From the standpoint of obtaining mechanical properties of the obtained cured body, furthermore, it is desired that the inorganic filler material has an average particle size of not less than 0.1 $\mu$m.

The average particle size of the inorganic filler material states a median diameter expressed by a volume percentage measured by using a particle size distribution analyzer based on the principle of the laser diffraction/scattering method.

Representative examples of the inorganic filler are elements of the Groups I, II, III and IV of periodic table, transition metals or oxides thereof, halides thereof, sulfates thereof or double salts thereof, and a mixture thereof can also be used as the inorganic filler. Preferably, there can be used oxides or composite oxides of metals such as silicon, titanium, aluminum, zirconium or tin. Composite oxides of these metals may, further, contain alkaline metals or alkaline earth metals such as sodium, potassium, magnesium and calcium.

Though there is no particular limitation on the shape of particles of the inorganic filler, it is desired that the particles have a spherical shape from the standpoint of realizing high surface gloss of the cured body obtaining esthetics.

The inorganic filler that is particularly preferably used in the present invention includes silica, a composite oxide or a clay mineral containing silicon as a constituent element, or various silicates (hereinafter they are called silica type fillers). The silica type fillers have excellent chemical stability and can be easily treated for their surfaces with a silane coupling agent.

Described below are concrete examples of the silica type fillers.

Silica such as quartz, precipitated silica, fumed silica, and sol-gel silica;

Composite oxides such as silica-titania, silia-zirconia, silica-barium oxide, silica-lanthania, silica-alumina, silica-calcia, silica-strontium oxide, silica-magnesia, silica-titania-sodium oxide, silica-titania-potassium oxide, silic-zirconia-sodium oxide, silica-zirconia-potassium oxide, silica-alumina-sodium oxide and silica-alumina-potassium oxide; and

Clay minerals or silicates such as talc, montmorillonite, zeolite and calcium silicate.

From the standpoint of good X-ray contrast, further, there can be favorably used oxides or fluorides of lanthanoid or yttrium, such as ytterbium oxide, ytterbium fluoride and yttrium fluoride.

There can be, further, used cation-eluting inorganic fillers such as silicate glass and fluoroaluminosilicate glass.

In the present invention, in particular, the silica composite oxide as represented by silica-zirconia or silica-titania is most desirably used as the inorganic filler component (B) from the standpoint of not only its excellent X-ray contrast but also the easiness of adjusting the refractive index depending on the content of the silica component and of satisfying specific conditions.

Further, upon being treated for its surfaces with a surface treating agent such as silane coupling agent, the above inorganic filler component (B) exhibits improved mixability to the polymerizable monomers contributing to improving mechanical strength and water-resisting property of the obtained cured body. Examples of the silane coupling agent include:

> Methyltrimethoxysilane,
> Methyltriethoxysilane,
> Methyltrichlorosilane,
> Dimethyldichlorosilane,
> Trimethylchlorosilane,
> Vinyltrichlorosilane,
> Vinyltriethoxysilane,
> Vinyltris($\beta$-methoxyethoxy) silane,
> $\gamma$-Methacryloyloxypropyltrimethoxysilane,
> $\gamma$-Chloropropyltrimethoxysilane,
> $\gamma$-Glycidoxypropyltrimethoxysilane, and
> Hexamethyldisilazane.

In order for the photocurable composition to acquire a viscosity in a range suited for filling in the cavity, to suppress the contraction by polymerization at the time of curing and to improve mechanical properties of the cured body that is obtained, the above inorganic filler component (B) is used in an amount of 100 to 1500 parts by mass, preferably, 150 to 1000 parts by mass and, most preferably, 170 to 600 parts by mass per 100 parts by mass of the above-mentioned polymerizable monomer component (A) . If the inorganic filler component (B) is used in too small amounts, the contraction by polymerization increases at the time of curing and the cured body has decreased mechanical properties. If the inorganic filler component (B) is used in too large amounts, the photocurable composition acquires an increased viscosity making it difficult to fill the photocurable composition in the cavity.

<Selection of the polymerizable monomer component (A) and the inorganic filler component (B)>

In the present invention, it is important to so select the above-mentioned polymerizable monomer component (A) and the inorganic filler component (B) as to satisfy a condition (X1) represented by the following formulas (1a) and (1b);

$$nF - 0.005 < nM < nF + 0.005 \qquad (1a)$$

$$nF + 0.020 < nP < nF + 0.040 \qquad (1b)$$

wherein,

nM is a refractive index of the polymerizable monomer component (A) at 25°C,
nP is a refractive index at 25°C of a polymer obtained by polymerizing the polymerizable monomer component (A), and
nF is a refractive index of the inorganic filler component (B) at 25°C,

and, more preferably, to satisfy a condition (X2) represented by the following formulas (2a) and (2b):

$$nF - 0.005 < nM < nF + 0.003 \qquad (2a)$$

$$nF + 0.025 < nP < nF + 0.035 \qquad (2b)$$

wherein, nM, nP and nF are as defined above.
That is, the refractive index of the polymerizable monomer, usually, increases if it is polymerized. By utilizing this fact according to the present invention, the inorganic filler component (B) is so selected that the refractive index of the inorganic filler component (B) is in a region (the above formula (1a) or (2a)) which is very close to the refractive index (nM) of the polymerizable monomer component (A) and is in a region (the above formula (1b) or (2b)) which is suitably separated away from the refractive index of a polymer obtained by polymerizing the polymerizable monomer component (A) under a predetermined condition. This makes it possible to assure the handling for filling/restoration, to bring the appearance of the cured body formed in the cavity close to that of the natural teeth so as to be in match with the natural teeth yet preventing esthetics from being deteriorated by the restoration of the cavity.
For example, the formula (1a) in the above condition (X1) or the formula (2a) in the condition (X2) can be rewritten as expressed by the following formula (1a') or (2a').

$$-0.005 < nM - nF < 0.005 \qquad (1a')$$

$$-0.005 < nM - nF < 0.003 \qquad (2a')$$

It will, therefore, be learned that the formula (1a) or the formula (2a) is expressing that the refractive index of the filler component (B) that is used is in a range very close to the refractive index of the polymerizable monomer component (A). In the photocurable composition containing the polymerizable monomer component (A) and the inorganic filler component (B), therefore, the light diffuses, reflects or scatters very little on the interface between the polymerizable monomer component (A) and the inorganic filler component (B) realizing, therefore, a large transmission of light and a large depth of curing. That is, as will be described in detail in Examples appearing later, the depth of curing is measured after the irradiation with light of a quantity of 500 mW/cm$^3$ for 30 seconds by using a halogen type dental curing light. The present invention makes it possible to attain the depth of curing thus measured of not less than 6 mm, specifically, not less than 8 mm and, further, not less than 10 mm and, therefore, to easily carry out the filling/restoration in a short period of time despite the cavity is deeply formed in the posterior teeth. That is, the work for filling the cavity with the photocurable composition and the work for curing the composition by the irradiation with light, can now be completed in one time or in a small number of times though they had so far been done for every time of filling.
If, for example, the refractive index of the inorganic filler component (B) is outside the range of the above formula (1a) (or (2a)), the depth of curing decreases, and the deep cavity in the posterior tooth cannot be filled unless the curing work is repeated many times for filling with the photocurable composition and irradiating it with light. Therefore, the efficiency of the filling/restoration decreases considerably.

Further, the formula (1b) in the above condition (X1) or the formula (2b) in the condition (X2) can be rewritten as expressed by the following formula (1b') or (2b').

$$0.020 < nP - nF < 0.040 \qquad (1b')$$

$$0.025 < nP - nF < 0.035 \qquad (2b')$$

It will, therefore, be learned that the formula (1b) or the formula (2b) is expressing that the refractive index of the filler component (B) that is used is in a region suitably separated away from the refractive index of the polymer of the polymerizable monomer component (A). That is, the polymer by itself that is obtained from the polymerizable monomer component (A) has high light transmittance as described earlier. However, since the refractive index of the polymer is suitably separated away from the refractive index of the inorganic filler component (B), the light diffuses, reflects and scatters to a large extent on the surface between the above two components in the cured body that is obtained from the photocurable composition. As a result, the cured body becomes translucent and exhibits appearance that is in match with the appearance of the natural teeth.

If, for example, the condition represented by the above formula (1b) (or (2b)) is not satisfied, then the cured body that is obtained becomes highly transparent and its appearance is not in match with that of the natural teeth and can no longer assure esthetics. Or even if the cured body may assure translucency, the condition represented by the above-mentioned formula (1a) (or 2a) is not satisfied and, therefore, the depth of curing becomes small decreasing the efficiency of handling for filling/restoration.

The polymerizable monomer component (A) is, usually, made up of a plurality of kinds of the polymerizable monomer components and, their combinations are adjusted so that the mechanical properties (strength, water-resisting property, etc.) of the obtained cured body and its adhesiveness to the teeth are within desirable ranges.

For instance, as described earlier, not less than 60% by mass and, specifically, not less than 70% by mass of the component (A) is the polyfunctional polymerizable monomer which is made up of a plurality of kinds of the polyfunctional polymerizable monomers such as those, usually, having an aliphatic group, an aromatic group and an acid group.

The component (A) that is made up of the plurality of kinds of the polymerizable monomers as described above is found for its refractive index nM by measuring the polymerizable monomer components that are really mixed up together according to a method that will be described in Examples appearing later. Here, the rule of addition holds and the reflective indexes of the individual polymerizable monomers are added up depending on the ratio of their amounts to find the refractive index nM of the component (A).

Further, if the component (A) is made up of the plurality of kinds of the polymerizable monomers, the refractive index nP of the polymer (i.e., copolymer) obtained from the component (A), if strictly speaking, must be found by really polymerizing the component (A) according to the method described in Examples appearing later. Here, the polymerizing conditions for obtaining the polymer for measuring the refractive index nP are nearly the same as the polymerizing conditions for curing the photocurable composition by filling the cavity formed in the tooth with the photocurable composition. Therefore, the monomers have been fully polymerized in the polymer and have been incorporated in the cured body. The rule of addition holds to a certain degree for the refractive index nP of the polymer (copolymer), too. Upon adding up the refractive indexes of the polymer measured for each of the monomers depending on their ratio of amounts, therefore, an approximate value of the refractive index nP of the polymer obtained from the components (A) can be calculated and can be used to design their blending ratios.

The inorganic filler component (B), too, may be made up of a plurality of kinds of the components. The rule of addition holds for the refractive index of this case, too. Upon adding up the refractive indexes of the individual inorganic filers depending on their ratio of amounts, therefore, it is allowed to calculate the refractive index nF of the inorganic filler component (B).

Here, in using a plurality of inorganic fillers, if differences are too large in their refractive indexes, the curable composition that has not been cured may become opaque despite the inorganic filler component (B) as a whole may satisfy the condition (X1) or (X2), and the depth of curing may often become small. If the plurality of kinds of the inorganic fillers are used to reliably alleviate such inconvenience, it is desired that at least not less than 70% by mass, specifically, not less than 80% by mass and, most desirably, the whole of the inorganic filler satisfy the above-mentioned condition (X1) and, desirably, the condition (X2) with the refractive index nM of the polymerizable monomer component (A) and with the refractive index nP of the polymer as references.

The above-mentioned various inorganic fillers have refractive indexes lying, usually, in a range of 1.4 to 1.7. The refractive indexes, however, are subject to vary to some extent due to the surface treatment by using a silane coupling agent and the like. Even if there are used inorganic fillers of which the refractive indexes have been known, therefore, it is necessary to practically measure the refractive indexes if their surfaces are treated.

Further, if the above-mentioned cation-releasing filler is used as the inorganic filler, its refractive index must be measured in a state where cations have all been released from the filler. The filler works to improve mechanical properties of the cured body as the polymerizable monomer is polymerized in the presence of ionic crosslinking formed by the cations released when the filler is used in combination with the acid component such as the polymerizable monomer that contains an acid group like carboxylic acid group or phosphoric acid group. The refractive index of the filler is varied by the release of ions, besides, the filler is finished to release the cations at the start of polymerization. If the cation-releasing filler is used, therefore, its refractive index must be measured in a state where the cations have been released therefrom.

As the polymerizable monomer component (A) in the present invention, there are, usually, used a plurality of kinds of polymerizable monomers to adjust properties (mechanical properties and adhesiveness to the teeth) of the cured body. Here, it is desired to select the kinds and amounts of the polymerizable monomers so that the refractive index nM of the component (A) is in a range of, preferably, 1.46 to 1.60 and, more preferably, 1.48 to 1.55. That is, upon setting the refractive index nM in the range of 1.48 to 1.55, it is allowed to set the refractive index nP of the polymer obtained from the polymerizable monomer component (A) in a range of about 1.52 to 1.57. Here, the silica type filler and, specifically, the silica type composite oxide in the inorganic filler component (B) exhibits a refractive index that is in a range of about 1.46 to about 1.56 dependent on the content of the silica component. That is, upon setting the refractive index of the polymerizable monomer component (A) to lie in the above-mentioned range, the inorganic filler component (B) can be easily selected so as to satisfy the above-mentioned condition (X1) or the condition (X2). Namely, there may be used a silica type composite oxide (e.g., silica titania or silica zirconia) that contains the silica component in a suitable amount.

Further, to adjust the refractive index nM to lie in the above-mentioned range by using the plurality of kinds of polymerizale monomers, in general, it is desired to select all of the plurality of kinds of the polymerizable monomers from the bifunctional (meth)acrylic compounds from the standpoint of satisfying the above-mentioned condition (X1) or (X2) and, at the same time, obtaining a cured body having a mechanical properties and the like properties so that it can be desirably used as a dental restorative material. For instance, trifunctional or more highly functional polymerizable monomers are capable of increasing the strength of the cured body but can be cured less deeply than the bifunctional polymerizable monomers. Though the reason is not clear, it is presumed that the crosslinking quickly takes place near the surface that is irradiated with light, and light reaches the bottom portion little. Further, the monofunctional polymerizable monomers tend to bring about a decrease in the strength of the cured body.

Further, if the plurality of kinds of bifunctional (meth)acrylic compounds are to be selected, it is desired to use, specifically, the aromatic bifunctional (meth)acrylic compounds and the aliphatic bifunctional (meth)acrylic compounds in combination. Use of the aromatic bifunctional (meth)acrylic compounds is advantageous in increasing the strength of the cured body while the aliphatic bifunctional (meth)acrylic compounds have relatively low viscosities. By using them in combination, therefore, it is allowed to adjust the viscosity of the polymerizable monomer components and to obtain a photocurable composition that can be favorably filled. Besides, a bifunctional (meth)acrylic compound having a functional group such as acid group is advantageous in improving adhesiveness to the teeth, and can be favorably added to the combination of the aromatic compounds and the aliphatic compounds.

In using the aromatic bifunctional (meth)acrylic compounds and the aliphatic bifunctional (meth)acrylic compounds in combination, in general, the aromatic bifunctional (meth) acrylic compounds have high nM and nP while the aliphatic bifunctional (meth)acrylic compounds have low nM and nP. By utilizing this, therefore, the amounts of these compounds can be set, and the refractive index nM can be set in the above-mentioned range.

Described below are specifically preferred examples of the bifunctional (meth)acrylic compound that can be used as the polymerizable monomer component (A) in the present invention.

Aromatic bifunctional (meth)acrylic compounds:

2,2-Bis[(3-methacryloyloxy-2-hydroxypropyloxy) phenyl] propane,

nM: 1.552

nP: 1.570

2,2-Bis(4-methacryloyloxypolyethoxyphenyl) propane,

nM: 1.540
nP: 1.567

Aliphatic bifunctional (meth)acrylic compounds:

Triethylene glycol dimethacrylate,

nM: 1.460
nP: 1.510

1,6-Bis(methacrylethyloxycarbonylamino)trimethyl hexane,

nM: 1.483
nP: 1.509

Described below are representative recipes of the polymerizable monomer component (A) using the above aromatic bifunctional (meth)acrylic compounds and the aliphatic bifunctional (meth)acrylic compounds in combination.

<Recipe 1>

Refractive index of a preparation (polymerizable monomer component (A)):

$$nM = 1.488 - 1.515$$

$$nP = 1.528 - 1.540$$

Recipe:

(A-1) 2,2-Bis[(3-methacryloyloxy-2-hydroxypropyloxy) phenyl] propane,
Bifunctional aromatic compound, nM: 1.552, nP: 1.570 30 - 50% by mass
(A-2) Triethylene glycol dimethacrylate,
Bifunctional aliphatic compound, nM: 1.460, nP: 1.510 10 to 30% by mass
(A-3) 1,6-Bis(methacrylethyoxycarbonylamino)trimethyl hexane,
Bifunctional aliphatic compound, nM: 1.483, nP: 1.509 20 - 60% by mass

For the polymerizable monomer component (A) of the recipe 1 using the above three kinds of bifunctional (meth)acrylic compounds (A-1) to (A-3), for example, there can be used a silica-titania composite oxide containing silica in an amount of 85 to 95% by mass and titania in an amount of 5 to 15% by mass (nF: 1.490 - 1.520) to satisfy the above-mentioned condition (X1) and, preferably, the condition (X2).

<Recipe 2>

Refractive index of a preparation (polymerizable monomer component (A)):

$$nM = 1.501 - 1.543$$

$$nP = 1.537 - 1.564$$

Recipe:

(A-1) 2,2-Bis[(3-methacryloyloxy-2-hydroxypropyloxy) phenyl] propane,
Bifunctional aromatic compound, nM: 1.552, nP: 1.570 45 - 90% by mass
(A-2) Triethylene glycol dimethacrylate,
Bifunctional aliphatic compound, nM: 1.460, nP: 1.510 10 to 55% by mass

For the polymerizable monomer component (A) of the recipe 2 using the above two kinds of bifunctional (meth)acrylic compounds (A-1) and (A-2), for example, there can be used a silica-zirconia composite oxide containing silica in an amount of 70 to 95% by mass and zirconia in an amount of 5 to 30% by mass (nF: 1.500 - 1.545) to satisfy the above-mentioned condition (X1) and, preferably, the condition (X2).

<Recipe 3>

Refractive index of a preparation (polymerizable monomer component (A)):

$$nM = 1.516 - 1.524$$

$$nP = 1.550 - 1.556$$

Recipe:

(A-1) 2,2-Bis(4-methacryloyloxypolyethoxyphenyl) propane,
Bifunctional aromatic compound, nM: 1.540, nP: 1.567 70 - 80% by mass
(A-2) Triethylene glycol dimethacrylate,
Bifunctional aliphatic compound, nM: 1.460, nP: 1.510 20 to 30% by mass

For the polymerizable monomer component (A) of the recipe 3 using the above two kinds of bifunctional (meth)acrylic compounds, for example, there can be used a silica-zirconia composite oxide containing silica in an amount of 75 to 85% by mass and zirconia in an amount of 15 to 25% by mass (nF: 1.510 - 1.530) to satisfy the above-mentioned condition (X1) and, preferably, the condition (X2).

<Recipe 4>

Refractive index of a preparation (polymerizable monomer component (A)):

$$nM = 1.520 - 1.540$$

$$nP = 1.551 - 1.563$$

Recipe:

(A-1) 2,2-Bis[(3-methacryloyloxy-2-hydroxypropyloxy) phenyl] propane,
Bifunctional aromatic compound, nM: 1.540, nP: 1.567
(A-2) 2,2-Bis(4-methacryloyloxypolyethoxyphenyl) propane,
Bifunctional aromatic compound, nM: 1.540, nP: 1.567
(A-3) Triethylene glycol dimethacrylate,
Bifunctional aliphatic compound, nM: 1.460, nP: 1.510

For the polymerizable monomer component (A) of the recipe 4 using the above three kinds of bifunctional (meth)acrylic compounds, for example,there can be used a silica-zirconia composite oxide containing silica in an amount of 70 to 80% by mass and zirconia in an amount of 20 to 30% by mass (nF: 1.520 - 1.545) to satisfy the above-mentioned condition (X1) and, preferably, the condition (X2).

In the above recipes 1 to 4, too, it is possible to adjust properties of the cured bodies by using other polymerizable monomers (e.g., monofunctional compounds and trifunctional or more highly functional compounds, or polymerizable monomers having a polar group such as acid group) in small amounts (e.g., not more than 40% by mass) on condition that the condition (X1) or, preferably, the condition (X2) is satisfied.

<Photo polymerization initiator (C)>

In the dental restorative material (photocurable composition) of the present invention, the photo polymerization initiator (C) is a component to polymerize and cure the above-mentioned polymerizable monomer component (A) by the irradiation with light. The wavelength of light irradiated for polymerization and curing is, usually, in a region of visible light from the standpoint of safety to the human body. Therefore, the photo polymerization initiator that is used has an excitation absorption wavelength region and, specifically, a maximum excitation absorption wavelength region in the region of visible light of 380 to 500 nm (preferably, 400 to 500 nm).

As the photo polymerization initiator, there is selected any compound that has been known per se. depending on the polymerization mechanism of the polymerizable monomer component (A) that is used. For the radically polymerizable monomer such as the above-mentioned (meth)acrylic compound, there is used a photo radical generator. For the cationically polymerizable monomer, there is used a known photo acid generator.

Described below are concrete examples of the photo radical generator.

$\alpha$-Diketones:

Camphorquinone, 1-phenyl-1,2-propanedione, etc. Bisacylphosphineoxides:

Bis(2,4,6-trimethylbenzoyl)-phenylphosphinoxide,
2,4,6-Trimethylbenzoyldiphenylphosphinoxide, etc.

$\alpha$-Aminoalkylphenones:

2-Benzyl-2-dimethylamino-1-(4-morpholinophenyl)-butanone-1,
2-Methyl-1-(4-metylthiophenyl)-2-morpholinopropane-1-one, etc.

Titanocenes:

Titanocene compounds such as bis($\eta$-5-2,4-cyclopentadiene-1-il)-bis(2,6-difluoro-3-(1H-pyrrole-1-il)phenyl)titanium, etc.

Among the above photo radical generators in the present invention, $\alpha$-diketones and bisacylphosphinoxides can be preferably used, and camphorquinone and 2,4,6-trimethylbenzoyldiphenylphosphinoxide can be more preferably used from the standpoint of polymerizing activity and little harm to the human body. Here, the camphorquinone has a maximum excitation absorption wavelength of 470 nm and the 2,4,6-trimethylbenzoyldiphenylphosphinoxide has a maximum excitation absorption wavelength of 380 nm.

The photo radical generators used as the polymerization initiator can be used in a single kind or, as required, in a combination of a plurality of kinds.

The photo polymerization initiator may be used in a so-called effective amount or, concretely, is used at a ratio of 0.01 to 30 parts by mass and, specifically, 0.1 to 5 parts by mass per 100 parts by mass of the polymerizable monomer component (A). Further, the photo polymerization initiator is used most desirably at a ratio of 0.1 to 1 part by mass per 100 parts by mass of the polymerizable monomer component (A) from the standpoint of not hindering the transmission of light by the polymerization initiator itself, assuring a large depth of curing and alleviating a decrease in the esthetics that is caused as the cured body is colored due to the color of the polymerization initiator itself.

To promote the polymerization, further, a reducing compound may also be used in combination with the above photo polymerization initiator.

As the reducing compound, there can be representatively used an aromatic tertiary amine. Though not limited thereto only, concrete examples thereof include:

4-Dimethylaminobenzoic acid,
Ethyl 4-dimethylamonobenzoate,
Lauryl 4-dimethylaminobenzoate,
3-Dimethylaminobenzoic acid,
Ethyl 3-dimethylaminobenzoate,
Dimethylamino-p-toluidine,
Diethylamino-p-toluidine,
p-Tolyldiethanolamine.

Among the above aromatic tertiary amines, 4-dimethylaminobenzoic acid and 4-dimethylaminobenzoic acid ester are preferably used.

The amount of the reducing compound that is added may vary depending on the kind of the polymerizable monomer component (A) used in combination or of any other components but is, usually, in a range of 0.001 to 20 mols and, specifically, 0.005 to 10 mols per mole of the photo polymerization initiator.

Further, as the photo acid generator used as the photo polymerization initiator, there can be preferably used diaryl iodonium salt compound, sulfonium salt compound, sulfonic ester compound, halomethyl-substituted S-triazine derivative and pyridinium salt compound and, specifically, diaryl iodonium salt compound and halomethyl-substituted S-triazine derivative.

The photo acid generator can be used in combination with the above photo radical generator in an amount of, for example, 0.001 to 20 mols and, specifically, 0.005 to 10 mols per mol of the photo radical generator.

<Coloring agent (D)>

The dental restorative material (photocurable composition) of the present invention can be blended with a coloring agent (D) to match the color tone of the cured body that is to be formed. That is, the coloring agent (D) is suitably used to adjust the color of the cured body that is formed being fitted in the cavity so that it exhibits a desired appearance (e.g., color tone of natural teeth or pure white like that of the whitening teeth).

The coloring agent may be a pigment or a dye, and either of them can be used in combination with those having different colors so that the cured body exhibits a desired color tone.

The pigment can be represented by an inorganic pigment. As the inorganic pigment, there can be exemplified titanium oxide, zinc oxide, zirconium oxide, zinc sulfate, barium sulfate, aluminum silicate, calcium silicate, carbon black, iron oxide, copper-chromite black, chrome oxide green, chrome green, violet, chrome yellow, lead chromate, lead molybdate, cadmium titanate, nickel-titanium yellow, ultramarine blue, cobalt blue, bismuth vanadate, cadmium yellow and cadmium red.

It is also allowable to use organic pigments such as monoazo pigment, diazo pigment, diazo condensed pigment, perylene pigment and anthraquinone pigment.

As the dye, there can be exemplified red dyes such as KAYASET RED G (Nihon Kayaku Co.), KAYASET RED B (Nihon Kayaku Co.), etc.; yellow dies such as KAYASET Yellow 2G, KAYASET Yellow GN, etc.; and blue dyes such as KAYASET Blue N, KAYASET Blue G, KAYASET Blue B, etc. By taking the stability of color tone in the oral cavity into consideration, however, it is desired to use water-insoluble pigments rather than water-soluble dyes.

Here, the coloring agents block the transmission of light and affect the depth of curing of the photocurable composition, as a matter of course. Specifically, in restoring a deep cavity, the restored part becomes dark and the esthetics deteriorates. In many cases, therefore, a white pigment (titanium oxide, zinc oxide, zirconium oxide, zinc sulfate, barium sulfate, aluminum silicate, etc.) is used as the coloring agent. However, the white pigments block the light to a high degree and cause the depth of curing to further decrease.

On the other hand, the photocurable composition of the present invention satisfies the above-mentioned condition (X1) and, preferably, the condition (X2) and, therefore, can be deeply cured; i.e., the depth of curing is little affected by the use of the coloring agent. Namely, despite the coloring agent is added, a sufficiently large depth of curing is maintained if the amount of its use is so small as to adjust the color tone of the cured body. Even in restoring a deep cavity, therefore, the filling/restoration can be favorably carried out. That is, according to the present invention, even in case the coloring agent is added, one time or a decreased number of times of work is enough for filling a large cavity with the photocurable composition and curing it by the irradiation with light effectively alleviating a decrease in the efficiency of handling for filling/restoration.

If further added, the photocurable composition of the present invention satisfies the above condition (X1) and, preferably, the condition (X2) and, therefore, the cured body thereof has a suitable translucency. Therefore, even without using any white pigment (or even using it in small amounts), the cured body, owing to its translucency, match with the natural teeth and exhibits excellent esthetics. That is, using quite no white pigment or using the white pigment in a small amount contributes to suppressing a decrease in the depth of curing caused by the coloring agent.

As described above, though the present invention is capable of suppressing a decrease in the depth of curing caused by the use of the coloring agent, there is a limitation on the degree of suppression. Therefore, the coloring agent should be added in an amount which is not more than a predetermined amount. Usually, to obtain a depth of curing suited for filling and restoring a deep cavity formed in the posterior tooth, the amount of the coloring agent is so suppressed that the contrast ratio is not more than about 0.30 and, specifically, not more than about 0.27 as measured for the photocurable composition of a thickness of 1 mm.

The contrast ratio is a scale of transparency found from stimulus values Y obtained by using a color difference meter, and is represented by a ratio (Yb/Yw) of a Y-value (Yb) on the black background and a Y-value (Yw) on the white background. If the contrast ratio is larger than the above range, it means that the coloring agent has been added in large amounts, the depth of curing of the photocurable composition is becoming small, and the efficiency of handling for filling/restoration is decreasing.

Further, it is desired that the amount of the coloring agent is so adjusted that the contrast ratio is not more than 0.55 and, specifically, in a range of 0.35 to 0.53 as measured for the photocurable composition in a state of the cured product thereof having a thickness of 1 mm. It has been said that a standard natural tooth (enamel of the labial surface of the anterior tooth) has the contrast ratio of about 0.45 (Didier Dietschi, DMD, PhD, A new shading concept based on natural tooth color applied to direct composite restorations, Quintessence Int. 2006; 37: 91-102) . The closer the contrast ratio of the cured body of the photocurable composition to the contrast ratio of the natural tooth, it is allowed to attain the matching with the appearance of the natural tooth. For instance, if the contrast ratio of the cured body of the photocurable composition is higher than the above range, the cured body buried in the cavity (restored portion of the tooth) acquires a too high degree of opaqueness and may appear in white color being distinct from the surrounding natural tooth. If the contrast ratio of the cured body is too low, on the other hand, the surrounding natural tooth appears whitish, and the cured body appears darkish developing mismatched appearance from the surrounding.

According to the present invention as described above, it is desired that the coloring agent is added in such an amount that the contrast ratio of the photocurable composition (uncured body) and the contrast ratio of the composition are both within predetermined ranges. The amount of addition of the coloring agent that satisfies the above ranges of contrast ratios is, usually, 0.001 to 80 ppm, specifically, 0.01 to 60 ppm and, most desirably, 0.05 to 40 ppm per the photocurable composition.

If a pigment is used as the coloring agent in the present invention, the pigment has an average particle size of, usually, not more than about 1 $\mu$m. If necessary, a commercially available pigment can be finely pulverized to acquire small particle sizes. For easy mixing with other components, further, the pigment can be added in the form of a dispersion to the blend. For example, the pigment can be used as a master batch being dispersed in a lowly viscous solution such as a reactive diluent or being dispersed in a powder such as of inorganic particles.

<Other components>

The photocurable composition of the present invention used as the dental restorative material can be, further, blended with any other known additives in addition to the above-mentioned components (A) to (D) within a range in which they do not impair the depth of curing or esthetics.

For instance, a polymerization inhibitor, an ultraviolet ray absorber and a viscosity-adjusting agent can be added as required.

In adding these components to the photocurable composition, it is desired that the amount of addition is so adjusted that the cured body thereof of a thickness of 1 mm has a contrast ratio of not more than 0.55 and, specifically, in a range of 0.35 to 0.53.

As the viscosity-adjusting agent, there often can be used a fine filler having a particle size of, for example, less than 0.07 $\mu$m and, specifically, in a range of 0.005 to 0.05 $\mu$m. It is desired that the fine filler is added in an amount of not more than 10 parts by mass and, specifically, not more than 5 parts by mass per 100 parts by mass of the inorganic filler component (B) so that the appearance which is obtained by the inorganic filler component (B) will not be impaired.

The dental restorative material of the present invention including the above photocurable composition is obtained, usually, by adding the above essential components and, as required, arbitrary components each in predetermined amounts together, mixing them together to a sufficient degree and, as required, removing bubbles from the obtained paste under reduced pressure.

The dental restorative material of the invention can be used for restoring any kinds of teeth. Specifically, the dental restorative material can be favorably used for restoring cavities formed in the posterior teeth in the same manner as usually using conventional composite resins for filling.

For instance, a cavity in a posterior tooth to be restored is treated with a suitable pretreating material or adhesive material, and the restorative material (photocurable composition) of the invention is filled therein and is formed into the shape of a tooth and polymerize and cure the restorative material by the irradiation with an intense light by using a dental curing light. That is, even in case a large cavity is formed in the posterior tooth, the restoration can be done by filling and curing the restorative material one time or a number of times close to one time. Therefore, the restorative material is suited for restoring, specifically, deep cavities of class I or class II. Further, the restorative material is best adapted for restoring a cavity of as deep as 3 to 6 mm. Restoration of such deep cavities can be completed in one time of polymerization or repeating the filling and photo polymerization a small number of times.

EXAMPLES

The present invention will now be described more concretely by way of Examples to which only, however, the invention is in no way limited.

In the following Examples and Comparative Examples, described below are how to measure the refractive index nM at 25°C of the polymerizable monomer (or the polymerizable monomer component (A) including a plurality of kinds of polymerizable monomers), the refractive index nP at 25°C of the polymer obtained by polymerizing the polymerizable monomer (or the polymerizable monomer component (A)) and the refractive index nF at 25°C of the inorganic filler.

<Refractive index nM of the polymerizable monomer>

The refractive index of the polymerizable monomer (or a mixture of the polymerizable monomers) that was used was measured by using the Abbe's refractometer (manufactured by Atago Co.) in a thermostatic chamber maintained at 25°C.

<Refractive index nP of the polymer>

As for the refractive index of the polymer of the polymerizable monomer (or a mixture of the polymerizable monomers) that was used, a polymer polymerized under nearly the same conditions as the polymerizing conditions in the cavity was measured by using the Abbe's refractometer (manufactured by Atago Co.) in the thermostatic chamber maintained at 25°C.

That is, a homogeneous polymerizable monomer (or a mixture of the polymerizable monomers) obtained by mixing together 0.2% by mass of CQ, 0.3% by mass of DMBE and 0.15% by mass of HQME was put into a mold having a hole measuring $\phi 7$ mm x 0.5 mm, and a polyester film was press-adhered onto both surfaces thereof. Thereafter, by using a halogen type dental curing light (Demetron LC, manufactured by Cybron Co.) of a light quantity of 500 mW/cm$^2$, the polymerizable monomer was irradiated with light for 30 seconds so as to be cured, and was then taken out from the mold to obtain a cured body of the polymerizable monomer. The refractive index was measured by setting the cured body onto the Abbe's refractometer (manufactured by Atago Co.) in which a solvent (bromonaphthalene) which does not dissolve the sample and has a refractive index higher than that of the sample was dropped on the sample in order to bring the cured body into close contact with the surface of measurement.

<Refractive index nF of the inorganic filler>

The refractive index of the inorganic filler (or a mixture of the inorganic fillers) that was used was measured by the immersion method by using the Abbe's refractometer (manufactured by Atago Co.).

That is, 1 g of the inorganic filler (or a mixture of the inorganic fillers) or a surface-treated product thereof was dispersed in 50 ml of anhydrous toluene in a 100-ml sampling bottle in the thermostatic chamber maintained at 25°C. While stirring the dispersion solution with a stirrer, a 1-bromotoluene was dropped therein little by little, the refractive index of the dispersion solution was measured at a moment when the dispersion solution became most clear, and the value that was obtained was regarded to be the refractive index thereof.

Described below are the polymerizable monomers, polymerization initiator and various additives used in Examples and Comparative Examples of the present invention. As for the polymerizable monomers, there are also described their refractive indexes nM and the refractive indexes nP of the polymers obtained by polymerizing the polymerizable monomers as described above.

[Polymerizable monomers]

bis-GMA;

2,2-Bis[(3-methacryloyloxy-2-hydroxypropyloxy) phenyl] propane
nM: 1.552
nP: 1.570

3G;

Triethylene glycol dimethacrylate
nM: 1.460
nP: 1.510

D-2.6E;

2,2-Bis(4-methacryloyloxypolyethoxyphenyl) propane

nM: 1.540
nP: 1.567

UDMA;

1,6-Bis(methacrylethyloxycarbonylamino) trimethylhexane
nM: 1.483
nP: 1.509

[Photo polymerization initiator]

CQ;
Camphorquinone

[Reducing compound (polymerization accelerator)]

DMBE;
N,N-Dimethyl p-ethyl benzoate

[Polymerization inhibitor]

HQME;
Hydroquinonemonomethyl ether

[Coloring agents]

Titanium oxide (white pigment)
Pigment Yellow 95 (yellow pigment)
Pigment Red 166 (red pigment)
Pigment Blue 60 (blue pigment)

[Preparation of the organic resin matrixes]

The above-mentioned polymerizable monomers were mixed together at mass ratios shown in Table 1 to prepare polymerizable monomer components (A) M-1 to M-9 that are to be used in Examples and Comparative Examples.
Table 1 also shows refractive indexes at 25°C of the polymerizable monomer components (A) and refractive indexes nP at 25°C of the polymers obtained from the monomer components (A) as measured according to the above-mentioned method.

Table 1

|  | Polymerizable monomer (mass %) | Refractive index nM (25°C) | Refractive index nP (25°C) |
| --- | --- | --- | --- |
| M-1 | bis-GMA(40)/3G(40)/UDMA(20) | 1.501 | 1.534 |
| M-2 | bis-GMA(47)/3G(53) | 1.503 | 1.538 |
| M-3 | bis-GMA(52)/3G(48) | 1.508 | 1.541 |
| M-4 | D2.6E(73)/3G(27) | 1.518 | 1.552 |
| M-5 | bis-GMA(30)/D2.6E(40)/3G(30) | 1.520 | 1.551 |
| M-6 | D2.6E(80)/3G(20) | 1.524 | 1.556 |
| M-7 | bis-GMA(30)/D2.6E(53)/3G(17) | 1.530 | 1.558 |
| M-8 | bis-GMA(70)/D2.6E(20)/3G(10) | 1.540 | 1.563 |
| M-9 | bis-GMA(90)/3G(10) | 1.543 | 1.564 |

[Inorganic fillers]

Table 2 shows the compositions, particle shapes, average particle sizes and refractive indexes nF at 25°C of the inorganic fillers F-1 to F-10 used in Examples and Comparative Examples.

The inorganic fillers other than the ytterbium fluoride of F-7 have been treated for their surfaces with a $\gamma$-methacryloyloxypropylmethoxysilane, and the refractive indexes are the values as measured in a state where their surfaces have been treated.

Table 2

|  | | Inorganic filler | Composition of inorganic filler (mass %) | Shape | Ave. primary particle size [$\mu$m] | Refractive index nF (25°C) |
|---|---|---|---|---|---|---|
| | F-1 | silica titania | $SiO_2$(90.8)/$TiO_2$(7.9)/$Na_2O$ (1.2) | spherical | 0.3 | 1.504 |
| | F-2 | silica zirconia | $SiO_2$(79.4)/$ZrO_2$(19.1)/$Na_2O$ (1.5) | spherical | 0.4 | 1.521 |
| | F-3 | silica zirconia | $SiO_2$(79.9)/$ZrO_2$(18.2)/$Na_2O$ (1.9) | amorphous | 3.0 | 1.526 |
| | F-4 | silica zirconia | $SiO_2$(72.9)/$ZrO_2$(25.5)/$Na_2O$ (1.6) | spherical | 0.2 | 1.542 |
| | F-5 | Silica zirconia | $SiO_2$(73.0)/$ZrO_2$(25.6)/$Na_2O$ (1.4) | amorphous | 3.0 | 1.543 |
| | F-6 | silica zirconia | $SiO_2$(80.1)/$ZrO_2$(18.7)/$Na_2O$ (1.2) | spherical | 0.07 | 1.519 |
| | F-7 | ytterbium fluoride | $YbF_3$ (purity of 99.5% or higher) | nearly spherical | 0.1 | 1.550 |
| | F-8 | F-2(70)/F-6(30) | | | | 1.52 |
| | F-9 | F-3(90)/F-7(10) | | | | 1.528 |
| | F-10 | F-2(28)/F-3(60)/F-6(12) | | | | 1.524 |

The photocurable compositions (dental restorative materials) prepared in Examples and Comparative Examples were measured by the methods described below.

Transparency (contrast ratio).

The photocurable composition pastes prepared in Examples and Comparative Examples were put into molds having a hole of 7 mm $\phi$ x 1 mm, and a polyester film was press-adhered onto both surfaces thereof.

By using a color-difference meter (TC-1800MK11, manufactured by Tokyo Denshoku Co.), measurement was taken on the black background and white background, Y-values (Yb and Yw) in tristimulus values were measured on the black background and white background, and contrast ratios were calculated according to the following formula while the photocurable composition pastes have not yet been cured.

By using the halogen type dental curing light (Demetron LC, manufactured by Cybron Co.) of a light quantity of 500 mW/cm$^2$, further, the photocurable composition pastes were irradiated on their both surfaces with light for 30 seconds so as to be cured and were, thereafter, taken out from the molds to take measurements in the same manner as above. The contrast ratios were calculated according to the following formula and were used as indexes of transparency.

The contrast ratio is an index of transparency that becomes opaque as it approaches 1.

```
Contrast ratio = Yb-value of when the background color
                 is black/Yw-value of when the background
                 color is white
```

Depth of curing.

The photocurable composition pastes (dental restorative materials) prepared in Examples and Comparative Examples were flown into metal molds of SUS of $\phi$4 mm x 10 mm thick, were covered with a 50-$\mu$m PET film from the upper side thereof, and excess of the pastes was pushed out.

Thereafter, by using the halogen type dental curing light (Demetron LC, manufactured by Cybron Co.) of a light quantity of 500 mW/cm$^2$, the photocurable composition pastes were irradiated with light for 30 seconds so as to be cured. The cured bodies were taken out therefrom, the unpolymerized pastes were removed by using a plastic spatula, and the cured portions were measured for their thicknesses by using a micrometer to regard them as depths of curing.

To attain the curing to a sufficient degree even in the bottom of a deep cavity in the posterior tooth, it is considered in a clinical sense that the depth of curing could have to be twice as depth of filling cavity in the clinic. Concretely speaking, if the paste is filled in a thickness of not less than 3 mm, then the depth of curing could have to be not less than 6 mm. More preferably, to fill the paste in a thickness of not less than 4 mm, the depth of curing could have to be not less than 8 mm.

Evaluating the color tone adaptability.

The photocurable composition pastes (dental restorative materials) prepared in Examples and Comparative Examples were filled in the artificial posterior teeth having a mimic cavity (4 mm $\phi$ x 4 mm), and were irradiated with light for 30 seconds by using the halogen type dental curing light (Demetron LC, manufactured by Cybron Co.) of a light quantity of 500 mW/cm$^2$ so that the pastes were cured.

The samples including the obtained cured products were polished by using a polishing member (Soflex Superfine manufactured by 3MESPE Co.), and were evaluated for their color tone adaptability with the naked eye on the following basis.

◎ : Boundary was very little discernible between the tooth surface and the cured body (restorative material), and color tone adaptability was high.

○ : Boundary was little discernible between the tooth surface and the cured body (restorative material), and color tone adaptability was high.

×: Boundary was discernible between the tooth surface and the cured body (restorative material), and restored part was discernible (restored part was dark or appeared to be white).

<Example 1>

To the matrix M-1, there were added:
CQ (photo polymerization initiator): 0.2% by mass
DMBE (reducing compound): 0.3% by mass
HQME (polymerization inhibitor): 0.15% by mass to prepare a homogeneous polymerizable monomer component (A) .

Next, 200 parts by mass of the inorganic filler F-1 was weighed as the component (B) in a mortar and to which 100 parts by mass of the polymerizable monomer component (A) was gradually added in the illumination of red light, and was mixed sufficiently in a dark place and was obtain a homogeneous paste thereof. The paste was then defoamed under reduced pressure to remove bubbles to thereby obtain a photocurable composition (dental restorative material).

The obtained photocurable composition was evaluated for various properties based on the methods mentioned above. Table 3 shows the photocurable composition and evaluated results thereof.

Table 3 also shows a difference in the refractive index (nM - nF) between the refractive index nM of the polymerizable monomer component (A) and the refractive index nF of the inorganic filler (B), and a difference in the refractive index (nP - nF) between the refractive index nP of a polymer obtained from the polymerizable monomer component (A) and the refractive index nF of the inorganic filler (B).

<Examples 2 to 11>

Photocurable compositions were prepared in the same manner as in Example 1 but changing the polymerizable monomer component (A) and the inorganic filler of the component (B) as shown in Table 3, and were evaluated for their properties. Table 3 shows the obtained photocurable compositions, evaluated results thereof, and differences in the refractive index (nM - nF), (nP - nF).

<Examples 12 to 14>

Photocurable compositions were prepared in the same manner as in Example 1 but changing the polymerizable monomer component (A) and the kind and amount of the inorganic filler of the component (B) as shown in Table 3, and were evaluated for their properties. Table 3 shows the obtained photocurable compositions, evaluated results thereof, and differences in the refractive index (nM - nF), (nP - nF).

Table 3

| | Polyerizable monomer component (A) | | | Inorganic filler (B) | | | | | | | |
|---|---|---|---|---|---|---|---|---|---|---|---|
| | *1 | *2 | *3 | *4 | *5 | nM -nF | nP -nF | *6 | *7 | *8 | *9 |
| Ex. 1 | M-1 (100) | 1.501 | 1.534 | F-1 (200) | 1.504 | -0.003 | 0.030 | 0.250 | 0.453 | 10< | ◎ |
| Ex. 2 | M-2 (100) | 1.503 | 1.538 | F-1 (200) | 1.504 | -0.001 | 0.034 | 0.198 | 0.512 | 10< | ◎ |
| Ex. 3 | M-3 (100) | 1.508 | 1.541 | F-1 (200) | 1.504 | 0.004 | 0.037 | 0.293 | 0.542 | 6.9 | ○ |
| Ex. 4 | M-4 (100) | 1.518 | 1.552 | F-2 (200) | 1.521 | -0.003 | 0.031 | 0.241 | 0.445 | 10< | ◎ |
| Ex. 5 | M-5 (100) | 1.520 | 1.551 | F-2 (200) | 1.521 | -0.001 | 0.030 | 0.184 | 0.431 | 10< | ◎ |
| Ex. 6 | M-6 (100) | 1.524 | 1.556 | F-2 (200) | 1.521 | 0.003 | 0.035 | 0.276 | 0.535 | 7.8 | ○ |
| Ex. 7 | M-6 (100) | 1.524 | 1.556 | F-3 (200) | 1.526 | -0.002 | 0.030 | 0.192 | 0.434 | 10< | ◎ |
| | Polyerizable monomercomponent (A) | | | Inorganic filler (B) | | | | | | | |
| | *1 | *2 | *3 | *4 | *5 | nM -nF | nP -nF | *6 | *7 | *8 | *9 |
| Ex. 8 | M-7 (100) | 1.530 | 1.558 | F-3 (200) | 1.526 | 0.004 | 0.032 | 0.287 | 0.519 | 7.1 | ◎ |
| Ex. 9 | M-8 (100) | 1.540 | 1.563 | F-4 (200) | 1.542 | -0.002 | 0.021 | 0.194 | 0.357 | 10< | ○ |
| Ex. 10 | M-9 (100) | 1.543 | 1.564 | F-4 (200) | 1.542 | 0.001 | 0.022 | 0.189 | 0.362 | 10< | ○ |
| Ex. 11 | M-9 (100) | 1.543 | 1.564 | F-5 (200) | 1.543 | 0.000 | 0.021 | 0.171 | 0.359 | 10< | ○ |
| Ex. 12 | M-4 (100) | 1.518 | 1.552 | F-8 (250) | 1.520 | -0.002 | 0.032 | 0.227 | 0.522 | 10< | ◎ |
| Ex. 13 | M-7 (100) | 1.530 | 1.558 | F-9 (300) | 1.528 | 0.002 | 0.030 | 0.251 | 0.516 | 10< | ◎ |
| Ex. 14 | M-5 (100) | 1.520 | 1.551 | F-10 (250) | 1.524 | -0.004 | 0.027 | 0.259 | 0.421 | 10< | ◎ |

*1: Amount (mass pts), *2: Refractive index (nM), *3: Refractive index (nP), *4: Amount (mass pts), *5: Refractive index (nF), *6: Contrast ratio of composition, *7: Contrast ratio of cured body, *8: Depth of curing [mm], *9: Color tone adaptability

As will be understood from the results of Examples 1 to 14, if the relationships of the refractive indexes nM, nP and nF are satisfying the condition (X1) and, further, the condition (X2) specified by the present invention, then the photocurable composition assumes a higher transparency and can be cured more deeply. Moreover, the cured body that is obtained has a suitable translucency enabling the color tone to be favorably adapted to the teeth. Specifically, in Examples 1, 2, 4, 5, 7 and 9 to 14 in which the contrast ratios before the polymerization are not more than 0.27, there are attained particularly large depths of curing of not less than 8 mm and all of which exhibiting favorable color tone adaptability.

<Comparative Examples 1 to 6>

Photocurable compositions were prepared in the same manner as in Example 1 but changing the polymerizable monomer component (A) and the inorganic filler of the component (B) as shown in Table 4, and were evaluated for their properties. Table 4 shows the obtained photocurable compositions, evaluated results thereof, and differences in the refractive index (nM - nF), (nP - nF).

Table 4

| | | Polyerizable monomer component (A) | | | Inorganic filler (B) | | | | | | | | |
|---|---|---|---|---|---|---|---|---|---|---|---|---|---|
| | | *1 | *2 | *3 | *4 | *5 | nM -nF | nP -nF | *6 | *7 | *8 | *9 |
| Comp. Ex. 1 | | M-1 (100) | 1.501 | 1.534 | F-2 (200) | 1.521 | -0.020 | 0.013 | 0.358 | 0.348 | 4.1 | × |
| Comp. Ex. 2 | | M-3 (100) | 1.508 | 1.541 | F-2 (200) | 1.521 | -0.013 | 0.020 | 0.341 | 0.352 | 4.7 | × |
| Comp. Ex. 3 | | M-7 (100) | 1.530 | 1.558 | F-2 (200) | 1.521 | 0.009 | 0.037 | 0.329 | 0.549 | 5.4 | ○ |
| Comp. Ex. 4 | | M-8 (100) | 1.540 | 1.563 | F-2 (200) | 1.521 | 0.019 | 0.042 | 0.355 | 0.589 | 4.3 | × |
| Comp. Ex. 5 | | M-4 (100) | 1.518 | 1.552 | F-3 (200) | 1.526 | -0.008 | 0.026 | 0.332 | 0.391 | 5.2 | ◎ |
| Comp. Ex. 6 | | M-8 (100) | 1.540 | 1.563 | F-3 (200) | 1.526 | 0.014 | 0.037 | 0.349 | 0.542 | 4.5 | ○ |

*1: Amount (mass pts), *2: Refractive index (nM),
*3: Refractive index of cured body (nP),
*4: Amount (mass pts), *5: Refractive index (nF),
*6: Contrast ratio of composition, *7: Contrast ratio of cured body,
*8: Depth of curing [mm], *9: Color tone adaptability

As will be understood from Comparative Examples 1 to 6, if the relationships of the refractive indexes nM, nP and nF are not satisfying the condition (X1) specified by the present invention, the contrast ratios before the polymerization become not less than 0.3 and the depths of curing become small.

<Examples 15 to 18, Reference Examples 1 and 2>

To the photocurable composition pastes obtained in Examples 2 and 4, there were added coloring agents shown in Table 5, and the mixtures were sufficiently kneaded in a dark place. The colored pastes were defoamed under reduced pressure to remove bubbles, and there were obtained photocurable compositions containing coloring agents.
The obtained photocurable compositions were evaluated for their properties based on the above-mentioned methods. Table 5 also shows the compositions and the evaluated results thereof.

Table 5

| | | (D) Coloring agents [ppm] | | | | | | | | |
|---|---|---|---|---|---|---|---|---|---|---|
| | Paste | White pigment | Yellow pigment | Red pigment | Blue pigment | Total | *1 | *2 | *3 | *4 |
| Ex. 15 | Ex. 2 | 0 | 10.0 | 3.0 | 1.3 | 14.3 | 0.203 | 0.519 | 10< | ◎ |
| Ex. 16 | Ex. 4 | 0 | 10.5 | 3.2 | 1.4 | 15.1 | 0.248 | 0.458 | 9.6 | ◎ |
| Ex. 17 | Ex. 2 | 30 | 10.0 | 3.0 | 1.3 | 44.3 | 0.237 | 0.548 | 8.9 | ○ |
| Ex. 18 | Ex. 4 | 20 | 10.5 | 3.2 | 1.4 | 35.1 | 0.294 | 0.547 | 6.2 | ○ |
| Ref. Ex. 1 | Ex. 2 | 118 | 0 | 0 | 0 | 118 | 0.410 | 0.651 | 4.2 | × |
| Ref. Ex. 2 | Ex. 4 | 85 | 0 | 0 | 0 | 85 | 0.482 | 0.707 | 2.8 | × |

*1: Contrast ratio of composition
*2: Contrast ratio of cured body
*3: Depth of curing [mm]
*4: Color tone adaptability

As will be understood from the results of Examples 15 to 18, the photocurable compositions blended with the coloring agents, too, could be cured sufficiently deeply if their contrast ratios were not more than 0.3 before the polymerization. Their color tone adaptabilities were favorable, too.

As will be understood from the results of Reference Examples 1 and 2, on the other hand, if the white pigment was contained in amounts larger than the range of the present invention, the contrast ratios before the polymerization became lager than 0.3, and the depths of curing were small. The cured bodies, too, possessed large contrast ratios and low color tone adaptabilities.

## Claims

1. A dental restorative material including a photocurable composition that contains a polymerizable monomer component (A), an inorganic filler component (B) having an average particle size of not smaller than 0.07 $\mu$m, and a photo polymerization initiator (C),
   said inorganic filler component (B) being contained in an amount of 100 to 1500 parts by mass per 100 parts by mass of said polymerizable monomer component (A), and
   said polymerizable monomer component (A) and said inorganic filler component (B) being so selected as to satisfy a condition (X1) represented by the following formulas (1a) and (1b) :

$$nF - 0.005 < nM < nF + 0.005 \qquad (1a)$$

$$nF + 0.020 < nP < nF + 0.040 \qquad (1b)$$

   wherein,

   nM is a refractive index of the polymerizable monomer component (A) at 25°C,
   nP is a refractive index at 25°C of a polymer obtained by polymerizing the polymerizable monomer component (A), and
   nF is a refractive index of the inorganic filler component (B) at 25°C;

   wherein the depth of curing is not less than 6 mm as measured after having been irradiated with light of a light quantity of 500 mW/cm$^2$ for 30 seconds by using a halogen-type dental curing light.

2. The dental restorative material according to claim 1, wherein said polymerizable monomer component (A) and said inorganic filler component (B) are so selected as to satisfy a condition (X2) represented by the following formulas (2a) and (2b):

$$nF - 0.005 < nM < nF + 0.003 \qquad (2a)$$

$$nF + 0.025 < nP < nF + 0.035 \qquad (2b)$$

   wherein, nM, nP and nF are as defined above.

3. The dental restorative material according to claim 1, wherein said polymerizable monomer component (A) contains a plurality of kinds of polyfunctional (meth)acrylic compounds and has a refractive index (at 25°C) in a range of 1.48 to 1.55.

4. The dental restorative material according to claim 3, wherein said plurality of kinds of polyfunctional (meth)acrylic compounds include a combination of polyfunctional aromatic (meth)acrylates and polyfunctional aliphatic (meth)acrylates.

5. The dental restorative material according to claim 4, wherein said polyfunctional aromatic (meth)acrylate is a 2,2-bis[(3-methacryloyloxy-2-hydroxypropyloxy)phenyl] propane and/or a 2,2-bis[(4-methacryloyloxypolyethoxyphenyl)]propane, and said polyfunctional aliphatic (meth) acrylate is a triethylene glycol dimethacrylate and/or a 1,6-bis (methacrylethyloxycarbonylamino) trimethylhexane.

6. The dental restorative material according to claim 3, wherein a silica composite oxide is used as said inorganic filler, and the content of the silica component in said composite oxide is so set as to satisfy said condition (X1).

7. The dental restorative material according to claim 1, wherein the dental restorative material is used for restoring cavities formed in the posterior teeth.

**Europäisches Patentamt**
**European Patent Office**
**Office européen des brevets**

## EUROPEAN SEARCH REPORT

Application Number

EP 18 19 9925

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/036582 A1 (YAMAKAWA JUNICHIRO [JP] ET AL) 20 February 2003 (2003-02-20) * paragraphs [0063], [0066], [0076], [0125] - [0126], [0141], [0147] - [0150], [0180]; examples 1-6 * | 1-7 | INV. A61K6/083 A61K6/00 |
| X | US 2011/046260 A1 (OKUBAYASHI MASAKI [JP] ET AL) 24 February 2011 (2011-02-24) * paragraphs [0105], [0135], [0139], [0150], [0160] - [0165]; claims 1-2, 14; examples 3-5; table 1 * | 1-7 | |
| X,D | JP S62 86003 A (TOKUYAMA SODA KK) 20 April 1987 (1987-04-20) * the whole document * | 1 | |
| A | DATABASE WPI Week 198845 Thomson Scientific, London, GB; AN 1988-318120 XP002754346, & JP S63 233905 A (KURARAY CO LTD) 29 September 1988 (1988-09-29) * abstract * | 1,7 | |

TECHNICAL FIELDS SEARCHED (IPC)

A61K

The present search report has been drawn up for all claims

| Place of search | Date of completion of the search | Examiner |
|---|---|---|
| Munich | 8 January 2019 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 19 9925

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2019

| Patent document cited in search report | | | Publication date | Patent family member(s) | | | Publication date |
|---|---|---|---|---|---|---|---|
| US 2003036582 | A1 | | 20-02-2003 | EP<br>US<br>WO | 1226807<br>2003036582<br>0215847 | A1<br>A1<br>A1 | 31-07-2002<br>20-02-2003<br>28-02-2002 |
| US 2011046260 | A1 | | 24-02-2011 | CA<br>CN<br>EP<br>JP<br>JP<br>US<br>WO | 2722661<br>102014848<br>2277495<br>5148696<br>WO2009133913<br>2011046260<br>2009133913 | A1<br>A<br>A1<br>B2<br>A1<br>A1<br>A1 | 05-11-2009<br>13-04-2011<br>26-01-2011<br>20-02-2013<br>01-09-2011<br>24-02-2011<br>05-11-2009 |
| JP S6286003 | A | | 20-04-1987 | JP<br>JP | H0447681<br>S6286003 | B2<br>A | 04-08-1992<br>20-04-1987 |
| JP S63233905 | A | | 29-09-1988 | JP<br>JP | H0791171<br>S63233905 | B2<br>A | 04-10-1995<br>29-09-1988 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009540107 T
- JP 2008502697 T
- JP 2004527602 T
- JP 2004149587 A
- JP 62086003 A
- WO 2007146239 A

**Non-patent literature cited in the description**

- **DIDIER DIETSCHI.** DMD, PhD, A new shading concept based on natural tooth color applied to direct composite restorations. *Quintessence Int.,* 2006, vol. 37, 91-102